# EUROPEAN PATENT APPLICATION

(11) **EP 4 371 551 A1**
(43) Date of publication of application: **22.05.2024**
(21) Application number: 22842522.9
(22) Date of filing: 15.07.2022
(51) Int. Cl.: A61K 8/63, A61K 8/73, A61Q 19/06, A61K 9/00, A61K 31/575, A61K 31/728, A61P 3/04

(54) **MOLECULAR ASSEMBLY OF BILE ACID OR BILE SALT AND PHARMACEUTICAL COMPOSITION COMPRISING SAME FOR REMOVING LOCAL FAT**

(30) Priority: 16.07.2021 KR 20210093764
(71) Applicant: Scai Therapeutics Co., Ltd., Seoul 06524 (KR)
(72) Inventor: KIM, Kyoung Hee, Daejeon 35246 (KR); KIM, Chul Hwan, Daejeon 35246 (KR); JANG, Ji Sung, Daejeon 34011 (KR)
(74) Representative: dompatent von Kreisler Selting Werner - Partnerschaft von Patent- und Rechtsanwälten mbB
(86) International application number: PCT/KR2022/010403
(87) International publication number: WO 2023/287260

(57) **Abstract**

The present invention relates to a molecular association of bile acid or bile salt and a composition comprising the same, and more specifically, to a skin permeable deoxycholic acid formulation which is the result of developing deoxycholic acid, a lipolytic pharmacological substance, as an external preparation instead of an existing injection, and thus remarkably improves patient compliance.

## Description

### [Technical Field]

The present invention relates to a molecular association of bile acid or bile salt and a pharmaceutical composition comprising the same for locally reducing fat, and more specifically, to a molecular association of bile acid or bile salt which is the result of developing bile acid or bile salt, a lipolytic pharmacological substance, as an external skin preparation instead of an existing injection, and thus remarkably improves patient compliance, and a pharmaceutical composition comprising the same for locally reducing fat.

### [Background Art]

Bile acid or bile salt can emulsify fat and promote the action of lipase, a digestive enzyme, to dissolve fatty acids. Since Bile acid or bile salt is also present in the digestive system of the human body, it can play a role in helping digestion and absorption by breaking down the ingested fat. Using this pharmacological mechanism, Allergan, a global company, developed a fat removal injection using deoxycholic acid, a type of bile acid, and developed Belkyra^{™} (in Canada). The Belkyra^{™} (in Canada) is delivered subcutaneously by injection to cause irreversible adipocyte destruction and promotes the production of new collagen in the treated area to improve the double chin, and it is an injection with less risk than liposuction. However, due to side effects such as pain, inflammation, bruises, swelling, contusion, and in severe cases, facial muscle weakness and jaw nerve damage, a professional medical person's procedure was required, and there was inconvenient that several visits to the hospital were required even after the procedure.

(Patent Document 1) KR 10-2061001 B1

### [Disclosure]

### [Technical Problem]

It is an object of the present invention to provide a molecular association having fat removal performance similar to that of a subcutaneous injection that apply skin permeable molecular association of bile acid or bile salt to the skin without using a surgical method of directly injecting and delivering the drug using a needle and syringe, and ensuring patient convenience without side effects of the injection.

In addition, it is an object of the present invention to provide a skin permeable molecular association of bile acid or bile salt, which is mass-produced, storage stability confirmed, and efficacy confirmed as a result of application to animals.

Further, it is an object of the present invention to provide a pharmaceutical composition for locally reducing fat that comprises the molecular association and is skin permeable.

### [Technical Solution]

The present invention provides a molecular association in which bile acid molecule or bile salt molecules are physically bonded, wherein when the molecular association is formed in a composition containing water, the molecular association has an associated structure in the composition.

Further, according to one embodiment of the present invention, the molecular association may have the pH of exceeding 8.5 and less than 10.

Further, according to one embodiment of the present invention, the average particle diameter of the molecular association may be 1.0 to 10 nm or less.

Further, according to one embodiment of the present invention, the molecular association may be amorphous.

Further, according to one embodiment of the present invention, the molecular association may have a concentration change rate of exceeding 1 and less than 10% for 12 months under 40±2°C/75±5% accelerated conditions.

Further, according to one embodiment of the present invention, the molecular association may have a particle size change rate of exceeding 1 and less than 10% for 12 months under 40±2°C/75±5% accelerated conditions.

Further, according to one embodiment of the present invention, the molecular association may have a pH change rate of exceeding 0 and less than 5% for 12 months under 40±2°C/75±5% accelerated conditions.

Further, according to one embodiment of the present invention, the bile acid may be any one from the group consisting of cholic acid, chenodeoxycholic acid, glycocholic acid, taurocholic acid, deoxycholic acid and lithocholic acid, and the bile salt may be any one salt from the group consisting of cholic acid, chenodeoxycholic acid, glycocholic acid, taurocholic acid, deoxycholic acid and lithocholic acid.

Further, according to one embodiment of the present invention, a pharmaceutical composition for locally reducing fat comprising the molecular association can be provided.

Further, according to one embodiment of the present invention, the composition may be applied and used as an external skin preparation, which is a non-surgical and no injection method.

Further, according to one embodiment of the present invention, the composition may further comprise at least one selected from the group consisting of glycerin, chia seed oil, glucan, hyaluronic acid, Lonicera Japonica (Honeysuckle) flower extract, collagen, ceramide, lecithin, betaine, trehalose, panthenol, squalane, caprylic/capric triglyceride, butylene glycol, propane diol, Pentylene glycol, Sodium Levulinate, Hydrogenated Lecithin, and Sodium Hyaluronate.

Further, according to one embodiment of the present invention, the composition may include the molecular association in an amount of 0.05 to 10% by weight.

Further, according to one embodiment of the present invention, the composition may be to treat a disease selected from the group consisting of obesity, fat redistribution syndrome, submental fat, a double chin caused by local fat accumulation, lower eyelid fat herniation formation, lipomas, Dercum's disease, lipodystrophy, buffalo hump dystrophy and combinations thereof.

Further, according to one embodiment of the present invention, the composition may be localized to a site selected from the group consisting of abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof.

Further, according to one embodiment of the present invention, the composition may be used in any one formulation from the group consisting of gel, cream, ointment, unguent, spray, thickened formulation and cataplasm.

Further, according to one embodiment of the present invention, the composition may have a bile acid concentration of 0.001 to 0.2 ug/ml measured in plasma 3 hours after application to the skin.

### [Advantageous Effects]

The present invention has the advantage of reducing the hassle of the administration method because it can be applied directly to the skin instead of a surgical method of directly injecting and delivering the drug using a needle and syringe. In addition, unlike existing product forms, it has the advantage of having an excellent effect on skin permeability, lipolysis and accumulation reduction despite not being an injection formulation.

In addition, since the skin gap is 80 nm, it is difficult for conventional pharmaceutical products to penetrate into the skin, but in the case of the present invention, since it has a small size of 0.5 to 5 nm, it has an effect of increasing skin permeability.

In addition, since nano-sized dispersed particles are easily exposed to aggregation or Ostwald ripening phenomenon, storage stability is low. On the other hand, the molecular association and composition of the present invention show excellent stability due to little change in properties, pH, and particle size at 40 ± 2°C/75 ± 5% acceleration conditions.

In addition, the present invention has the advantage of not necessarily using a third material such as surfactants, micelles, cyclodextrins, lipids, albumin, water-soluble polymers, stabilizers/dispersants, and carriers such as nanoparticles and porous particles that has been used in addition to API and water in order to improve solubility in the existing technology.

### [Description of Drawings]

Fig. 1 is the result of Zetasizer particle size analysis of the molecular association of deoxycholic acid.
Fig. 2 is a TEM image of the molecular association of deoxycholic acid.
Fig. 3 is a graph of the calibration curve of the molecular association of deoxycholic acid.
Fig. 4 is a diagram of preadipocyte cytotoxicity of the molecular association of deoxycholic acid.
Fig. 5 shows the process of preadipocyte differentiation into adipocytes.
Fig. 6 is a diagram of adipocyte cytotoxicity of the molecular association of deoxycholic acid.
Fig. 7 is a diagram of measuring the amount of skin permeation by collecting the subcutaneous tissue after single injection of deoxycholic acid precursor into the subcutaneous tissue of a live mouse and continuous application of the same amount of the deoxycholic acid precursor and the deoxycholic acid molecular association of the present invention on the skin, respectively.
Fig. 8 is a diagram of comparing the amount of skin permeation using the amount of distribution in the skin tissue by collecting the subcutaneous tissue after single injection of deoxycholic acid precursor into the subcutaneous tissue of a live mouse and continuous application of the same amount of the deoxycholic acid precursor and the deoxycholic acid molecular association of the present invention on the skin, respectively.
Fig. 9 is a diagram comparing the amount of plasma distribution of the precursor of deoxycholic acid and molecular association over time after a single subcutaneous injection of deoxycholic acid precursor into the skin of a live mouse and continuous application of the same amount of the precursor and molecular association of deoxycholic acid to the skin.
Fig. 10 is the H&E staining result for evaluation of histological effects of the precursor of deoxycholic acid and molecular association on the subcutaneous tissue according to the application time after a single subcutaneous injection of deoxycholic acid precursor into the skin of a live mouse and continuous application of the same amount of the precursor and molecular association of deoxycholic acid to the skin.
Fig. 11 is the result of Masson's Trichrome staining for evaluation of histological effects of the precursor of deoxycholic acid and molecular association on the subcutaneous tissue according to the application time after a single subcutaneous injection of deoxycholic acid precursor into the skin of a live mouse and continuous application of the same amount of the precursor and molecular association of deoxycholic acid to the skin, and shows the increase in the area of the part stained in blue due to the destruction of adipocytes or the increase in collagen.
Fig. 12 is a diagram comparing a body weight change with a negative control group when 1.0% and 2.5% of the deoxycholic acid molecular association were applied to the skin of obese mouse twice a day for 4 weeks (N=5).
Fig. 13 is a diagram comparing a waist girth change with a negative control group when 1.0% and 2.5% of the deoxycholic acid molecular association were applied to the skin of obese mouse twice a day for 4 weeks (N=5).
Fig. 14 is a diagram comparing a waist girth change with a negative control group when 1.0% and 2.5% of the deoxycholic acid molecular association were applied to the skin of obese mouse twice a day for 4 weeks.

### [Best Mode]

Poorly soluble drugs have extremely low saturated solubility in aqueous solution and extremely high interfacial tension/energy with water, so they are thermodynamically unstable, resulting in precipitation or phase separation. Therefore, in order to increase the drug content (i.e., solubility) in a pharmacologically meaningful way, a surface active agent capable of lowering the interface energy or a carrier capable of containing high content of drug was required.

In common, these existing solubility enhancement technologies either necessarily use the third material other than API and water or act as a key factor in improving solubility. For example, surfactants, micelles, cyclodextrins, lipids, albumin, water-soluble polymers, stabilizers/dispersants, nanoparticles, and porous particles fall under these third substances.

However, the inventors of the present invention prepare a molecular association utilizing polar interactions or hydrogen bonds so that the surface of the structure is hydrophobic without using the third material as above, thereby increasing the permeability to the phospholipid membrane, and adjust the particle diameter of the association to a level of 1.0 to 10 nm, thereby increasing the transmittance through skin gap having a size of about 80 nm. The inventors of the present invention have completed the present invention by confirming that the pharmacological substance is delivered to adipocytes in the skin through this to exhibit excellent effects on lipolysis and fat accumulation reduction.

It is explained in more detail below.

### Terms

In the present invention, the "bile acid" and "bile salt" means a steroid acid (and/or its carboxylic acid anion thereof), and salts thereof, which are found in the bile of animals (e.g., a human). Non-limiting examples thereof include any one bile acid selected from the group consisting of cholic acid, chenodeoxycholic acid, glycocholic acid, taurocholic acid, deoxycholic acid and lithocholic acid, or a bile salt thereof.

In the present invention, the "molecular association of bile acid or bile salt" refers to a molecular association prepared by applying shear stress to a solution containing bile acid so that bile acid molecule or bile salt molecules are physically bonded, and it means a structure in which bile acid molecules stick together.

In the present invention, the "bile acid molecule or bile salt molecules" refers to a precursor used to produce the molecular association of bile acid or bile salt according to the present invention, or the molecule itself of bile acid or bile salt as a precursor, and may also be referred to as "precursor". That is, the molecules of bile acid or bile salt according to the present invention refer to bile acid or bile salt to which shear stress is not applied.

In the present invention, the term "patient", "subject", "individual" and the like are used interchangeably herein, and refer to any animal, or cells thereof whether in vitro or in situ, amenable to the methods described herein. In certain non-limiting embodiments, the patient, subject or individual is a human.

In the present invention, the term "composition" or "pharmaceutical composition" refers to a mixture of at least one compound of the present invention with other chemical components, such as carriers, stabilizers, diluents, dispersing agents, suspending agents, thickening agents, and/or excipients. The pharmaceutical composition facilitates administration of the compound to an organism.

In the present invention, the term "effective amount," "pharmaceutically effective amount" and "therapeutically effective amount" refer to a nontoxic but sufficient amount of an agent to provide the desired biological result. That result may be reduction and/or alleviation of the signs, symptoms, or causes of a disease, or any other desired alteration of a biological system. An appropriate therapeutic amount in any individual case may be determined by those of ordinary skill in the art using routine experimentation.

In the present invention, the term "efficacy" refers to the maximal effect (Eₘₐₓ) achieved within an assay.

In the present invention, the term "treatment" or "treating" is defined as the application or administration of a therapeutic agent, i.e., a compound of the present invention (alone or in combination with another pharmaceutical agent), to a patient, or application or administration of a therapeutic agent to a tissue or cell line isolated from a patient (e.g., for diagnosis or ex vivo applications) with the purpose to cure, heal, alleviate, relieve, alter, remedy, ameliorate, improve or affect a condition contemplated herein, the symptoms of a condition contemplated herein or the potential to develop a condition contemplated herein. Such treatments may be specifically tailored or modified, based on knowledge obtained from the field of pharmacology.

In the present invention, "therapeutically effective amount" is an amount of the compound of the present invention, that when administered to a patient, ameliorates a symptom of the disease. The amount of the compound of the present invention which constitutes the "therapeutically effective amount" may vary depending on the compound, the disease state and its severity, the age of the patient to be treated, and the like. The therapeutically effective amount can be determined routinely by those of ordinary skill in the art in consideration of their knowledge and to the present disclosure.

In the present invention, the term "applying as an external skin application" refers to, for example, non-surgical and no injection method to apply a pharmaceutical composition to the outside of the skin to deliver the drug to the inside of the skin. In addition, it can be routinely determined by those skilled in the art in consideration of their knowledge and the present disclosure.

In the present invention, the term "locally reducing fat" means an action to treat a disease selected from the group consisting of, for example, obesity, fat redistribution syndrome, submental fat, a double chin caused by local fat accumulation, lower eyelid fat herniation formation, lipomas, Dercum's disease, lipodystrophy, buffalo hump dystrophy and combinations thereof. In addition, it can be routinely determined by those skilled in the art in consideration of their knowledge and the present disclosure.

In the present invention, the term "local fat" refers to localization to a site selected from the group consisting of, for example, abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof. In addition, it can be routinely determined by those skilled in the art in consideration of their knowledge and the present disclosure.

In the present invention, the term "external skin preparation" refers to, for example, a formulation that can be applied to the skin, and is used in any one formulation from the group consisting of gel, cream, ointment, unguent, spray, thickened formulation and cataplasm. In addition, it can be routinely determined by those skilled in the art in consideration of their knowledge and the present disclosure.

### Molecular association of bile acid or bile salt

The present invention provides a molecular association in which bile acid molecule or bile salt molecules are physically bonded, wherein when the molecular association is formed in a composition containing water, the molecular association has an associated structure in the composition.

In the present invention, the average particle diameter of the molecular association may be 1.0 to 10 nm or less, preferably 1.5 nm or more, 2.0 nm or more, and 7.0 nm or less, 5.0 nm or less, 3.0 nm or less. The average particle diameter of the molecular association can be measured through a diffraction experiment, preferably using a Zetasizer or Small Angle Neutron Scattering (SANS). Also, images can be obtained using Transmission Electron Microscopy. When the average particle diameter of the molecular association exceeds 10 nm, there are problems in dispersibility, transparency and transmittance. In addition, the lower limit of the average particle diameter of the molecular association is not particularly limited, but about 1.0 nm or more may be used.

As the molecular association according to the present invention is prepared by applying shear stress to bile acid molecule or bile salt molecules, it can have an amorphous shape even though it is manufactured in nano size, and accordingly, it is not only easy to adjust the size, but also has excellent skin permeability.

In addition, the molecular association according to the present invention may have the pH of exceeding 8.5 and less than 10. When the pH value satisfies the above range, the skin permeability of the molecular association is increased, and fat can be effectively decomposed after reaching adipocytes. In addition, the molecular association of the present invention may have a relatively high pH as it is used as a topical, unlike substances conventionally used as injections, and storage stability may be further improved as the pH is high. Specifically, the pH of the molecular association may be exceeding 8.6, exceeding 8.7, exceeding 8.8, exceeding 8.9, exceeding 9.0, exceeding 9.1, and less than 9.9, less than 9.8, less than 9.7, less than 9.6, less than 9.5, less than 9.4, less than 9.3.

Further, the molecular association according to the present invention has excellent storage stability. Unlike general nano-sized dispersed particles, which have low storage stability because they are easily exposed to aggregation or Ostwald ripening phenomenon, the molecular association and composition of the present invention have excellent stability due to little change in properties, pH, and particle size under accelerated conditions of 40 ± 2°C/75 ± 5%.

Specifically, the molecular association according to the present invention may have a concentration change rate of exceeding 1 and less than 10%, preferably exceeding 1 and less than 5%, more preferably exceeding 1 and less than 4% for 12 months under 40±2°C/75±5% accelerated conditions. In addition, the molecular association according to the present invention may have a concentration change rate of exceeding 1 and less than 10%, preferably exceeding 1 and less than 5%, more preferably exceeding 1 and less than 3% for 3 months under 40±2°C/75±5% accelerated conditions.

Specifically, the molecular association according to the present invention may have a particle size change rate of exceeding 1 and less than 10%, preferably exceeding 1 and less than 7%, more preferably exceeding 1 and less than 5% for 12 months under 40±2°C/75±5% accelerated conditions. In addition, the molecular association according to the present invention may have a particle size change rate of exceeding 1 and less than 10%, preferably exceeding 1 and less than 5%, more preferably exceeding 1 and less than 3% for 3 months under 40±2°C/75±5% accelerated conditions.

Specifically, the molecular association according to the present invention may have a pH change rate of exceeding 0 and less than 5%, preferably exceeding 0 and less than 3%, more preferably exceeding 0 and less than 1.5% for 12 months under 40±2°C/75±5% accelerated conditions. In addition, the molecular association according to the present invention may have a pH change rate of exceeding 0 and less than 5%, preferably exceeding 0 and less than 3%, more preferably exceeding 0 and less than 1.5% for 3 months under 40±2°C/75±5% accelerated conditions.

The change rate means an average rate of change obtained by calculating an average value of change rates for each month up to the period.

As described above, it can be seen that the stability of the molecular association according to the present invention is excellent.

### Method for manufacturing molecular association

The molecular association of bile acid or bile salt according to one embodiment of the present invention can be manufactured by applying shear stress to a solution containing the precursor of the molecular association, bile acid or bile salt.

The shear stress applied to the solution containing the precursor of the molecular association, bile acid or bile salt, may be either mechanical shear stress or ultrasonic application.

The mechanical shear stress may be applied by passing the solution through a silica-filled column or filter paper. Hereinafter, the mechanical shear stress will be described in detail.

According to one embodiment of the present invention, the mechanical shear stress may be applied by passing the solution containing the precursor of the molecular association, bile acid or bile salt, through a silica-filled column. When the solution containing bile acid or bile salt passes through a column filled with silica or the like, the precursor of the molecular association, bile acid or bile salt, is subjected to very high shear stress as it passes through a physically narrow area.

The silica may be spherical or prismatic, but its shape is not limited.

The average particle size of the silica may be 1.0 to 50 *µ*m, specifically 1.5 *µ*m or more, 2 *µ*m or more, and 40 *µ*m or less, 30*µ*m or less, 20 *µ*m or less, 10 *µ*m or less, 5 *µ*m or less. When the size of the silica is less than 1.0 um or exceeding 50 um, even if the solution containing bile acid or bile salt passes through the column filled with silica, shear stress is not applied and thus molecular association may not change.

A negative pressure of 0.1 bar to 1.0 bar or 0.2 bar to 0.9 bar may be applied to the bottom of the silica-filled column. When the negative pressure applied to the bottom of the silica-filled column is less than 0.1 bar, the time required for the solution containing bile acid or bile salt to pass through the column increases, and thus the manufacturing time of the molecular association of bile acid or bile salt according to the present invention may be delayed. In addition, when the negative pressure applied to the bottom of the silica-filled column exceeds 1.0 bar, the time required for the solution containing bile acid or bile salt to pass through the column is reduced, thereby reducing the manufacturing time of the molecular association of bile acid or bile salt according to the present invention, but the manufacturing cost may increase because additional pump equipment is required.

According to another embodiment of the present invention, the mechanical shear stress may be applied by passing the solution containing bile acid or bile salt through one or more filter papers. When passing through the one or more filter papers, the precursor of the molecular association, bile acid or bile salt is subjected to very high shear stress by passing through a physically narrow area.

The filter paper may be one filter paper or a plurality of filter papers of two or more. When the filter paper is a plurality of filter papers of two or more, the filter papers may be stacked. When the filter paper is a plurality of filter papers of two or more, shear stress higher than that of one filter paper may be provided.

The size of pores of the filter paper may be 0.1 to 5.0 micron or 0.3 to 4.5 micron. When the pore size of the filter paper is less than 0.1 micron, the amount of the bile acid-containing solution passed through or filtered through the filter paper is too small, and thus the manufacturing speed of the molecular association of bile acid or bile salt according to the present invention may be reduced. When the pore size of the filter paper exceeds 5.0 micron, the bile acid-containing solution simply passes through the filter paper, and shear stress may not be effectively applied.

The shear stress may be applied using ultrasonic waves. Hereinafter, application of ultrasonic waves will be described in detail.

According to one embodiment of the present invention, the shear stress may be applied by applying ultrasonic waves to the solution containing bile acid or bile salt.

When the ultrasonic waves are applied to the solution containing bile acid or bile salt, pressure waves are generated, and the shear stress may be applied to bile acid or bile salt, which is a precursor of the molecular association, by the pressure wave.

The intensity of the applied ultrasonic waves may be 200 J/sec to 800 J/sec or 400 J/sec to 600 J/sec.

The energy applied per volume of the applied ultrasonic waves may be calculated as the intensity of the ultrasonic waves (J/sec) × the applied time (sec) / the measured volume (ml).

According to one embodiment of the present invention, Energy applied per volume of ultrasonic waves applied to the solution containing bile acid or bile salt may be 100 J/ml to 90 kJ/ml.

When the energy of the ultrasonic waves is less than 100 J/ml, it may be difficult to form the molecular association because sufficient shear stress is not applied to the solution containing bile acid or bile salt. In addition, when the energy of the ultrasonic waves exceeds 90 kJ/ml, it may be difficult to form the molecular association because excessive heat is applied to the solution containing bile acid or bile salt.

The ultrasonic waves may be applied for 10 seconds to 60 minutes at 10°C to 80°C. When the ultrasonic waves are applied at a temperature of less than 10°C, there is no change in the solution containing bile acid or bile salt, and when applied at a temperature exceeding 80°C, a phase change occurs in the solution containing bile acid or bile salt and the molecular association of bile acid or bile salt according to the present invention can be difficult. In addition, when the ultrasonic waves are applied for less than 10 seconds, there is no change in the solution containing bile acid or bile salt. When the ultrasound is applied for a time period exceeding 60 minutes, the molecular association in the solution containing bile acid or bile salt is transformed, and the molecular association of bile acid or bile salt according to the present invention cannot be formed.

According to another embodiment of the present invention, as a method of applying the shear stress, a column filled with silica may be combined with an ultrasonic generator. The column filled with silica may be placed inside the ultrasonic generator, or the column filled with silica and the ultrasonic generator may be separated and continuously placed.

For example, after pouring the solution containing bile acid or bile salt into the column filled with silica, the column may be placed in the ultrasonic generator to apply ultrasonic waves. In addition, after ultrasonic waves are applied to the solution containing bile acid or bile salt, the solution may be passed through the column filled with silica.

### Pharmaceutical composition for locally reducing fat

The present invention provides a pharmaceutical composition for locally reducing fat comprising the molecular association.

In the present invention, the pharmaceutical composition for locally reducing fat can be used in any one formulation from the group consisting of gel, cream, ointment, unguent, spray, thickened formulation and cataplasm.

In the present invention, the pharmaceutical composition for locally reducing fat may further comprise at least one selected from the group consisting of glycerin, chia seed oil, glucan, hyaluronic acid, Lonicera Japonica (Honeysuckle) flower extract, collagen, ceramide, lecithin, betaine, trehalose, panthenol, squalane, caprylic/capric triglyceride, butylene glycol, propane diol, Pentylene glycol, Sodium Levulinate, Hydrogenated Lecithin, and Sodium Hyaluronate, in addition to the molecular association.

Further, in the present invention, in addition to the above ingredients, the pharmaceutical composition for locally reducing fat may further include any ingredient used in the art for use in a cream formulation without particular limitation.

In the present invention, the pharmaceutical composition for locally reducing fat may be included so that the molecular association is 0.05% by weight to 10.0% by weight, specifically 0.08% by weight or more, 0.1% by weight or more, 0.3% by weight or more, 0.5% by weight or more, 1.0% by weight or more, and 5% by weight or less, 3.0% by weight or less, 2.0% by weight or less, 1.0% by weight or less.

In the present invention, the pharmaceutical composition for locally reducing fat may have a concentration of 0.001 to 0.2 µg/ml, specifically 0.01 *µ*g/mℓ or more, 0.05 *µ*g/mℓ or more, 0.1 *µ*g/mℓ or more, and 0.18 *µ*g/mℓ or less, 0.15 *µ*g/mℓ or less, 0.12*µ*g/mℓ or less of bile acid or bile salt measured in plasma 3 hours after application to the skin.

Hereinafter, the present invention will be described in more detail through the Examples of the present invention. It goes without saying that the present invention is not limited to these examples.

### [Example]

### Example 1-1. Preparation of molecular association of deoxycholic acid(DCA)

3.6g of deoxycholic acid (DCA) was put into a 250ml beaker and dissolved in 177g of ethanol. 142 g of SYLOID 244 FP was put into a 3L beaker, and stirred at 50 rpm using an overhead stirrer. After gradually adding 17 g of 1M NaHCO₃ and 5 g of 1M Na₂SO₄ to the silica under stirring, the DCA solution was slowly added thereto and stirred for 30 minutes so that the added aqueous solution could be well deposited on the silica. 1780 g of ethanol was added to a 3L beaker, and the silica containing deoxycholic acid was slowly added thereto while stirring at 70 rpm. After completion of the addition, it was extracted for a sufficient time by further stirring for 45 minutes. After stirring was completed, it was sequentially filtered using a 1 um paper filter and a 0.45 um membrane filter. The filtered effluent was 1450 g, and concentrated to 704 g by partially removing ethanol using a rotary evaporator. 1640 g of water was put into a beaker, 0.3 g of NaHCO₃ was added, and the above primary concentrate was added thereto. This diluted solution was concentrated for 3 hours using a rotary evaporator to obtain 112 g of a colorless and transparent liquid. The concentration of this solution was 2.34% and the pH was 9.22. The recovery rate of DCA in this process was 69%.

### Example 1-2. Preparation of molecular association of deoxycholic acid(DCA)

A DCA solution was prepared by placing 2.0 g of deoxycholic acid (DCA) and 98.75 g of ethanol in a 500 mL beaker and stirring with a magnetic stirrer. 80 g of ethanol was added to 8.2 g of SYLOID 244 FP to sufficiently wet the silica. 1.3mL of a co-salt aqueous solution was prepared by mixing aqueous solutions of 1M NaHCO₃ and 1M Na₂SO₄ at a certain ratio. A 1.00 um paper filter was placed on a Buchner funnel, the paper filter was wetted with ethanol, and the filter was adsorbed to the bottom of the funnel using a pump, and then the prepared wet silica was slowly poured. When about 1 cm of ethanol remained on the packed silica, the co-salt aqueous solution and 100 g of purified water were slowly poured. Thereafter, 100 g of ethanol was additionally poured in, and after some of the ethanol was filtered, the pump was stopped, and the 1000 ml filter bottle below it was replaced. After operating the pump again, the prepared deoxycholic acid solution was slowly poured, and 202 g of ethanol was additionally poured. When the obtained effluent was filtered using a 0.45um membrane filter, 394.2 g of the filter filtrate was obtained. Using another 2.0 L beaker, 920 g of a 4 mM NaHCO₃ aqueous solution was prepared, and the filter filtrate was gradually added to dilute the mixture. This diluted liquid was concentrated for 2 hours and 25 minutes at 30°C and 180 rpm using a rotary evaporator. The amount of the final concentrate was 98.19 g, and a molecular association of deoxycholic acid with a concentration of 1.92% was obtained. The particle size was 1.36 nm, the pH was 9.15, and the final recovery was 94.2%.

### Example 1-3. Molecular association of Sodium deoxycholate(NaDC)

18.0 g of sodium deoxycholate (NaDC, Sigma Aldrich #30970) was put in a 1L beaker and dissolved in 604 g of water using a magnetic bar. Silica gel 60 (Merck) 60.5g was put into a 400mL beaker containing 242g of 0.1M NaHCO₃ and mixed with a spatula. Wetted silica was packed while slowly pouring it into a vacuum filtration device (200 mbar pressure) equipped with a vacuum pump. The NaDC solution was added to the packed silica. Before the silica bed dried, 160 g of water was added in two portions of 80 g each. The outflow time was 1 hour in total, and after the outflow was completed, it was filtered using a 0.45um membrane filter. The filtered effluent was 808g. The concentration of this solution was 2.07% and the pH was 7.67. The recovery rate of sodium deoxycholate in this process was 93%.

### Example 2. Composition containing molecular association of deoxycholic acid

Since Example 1 above is in an aqueous solution, it tends to flow when applied to the skin, so there is a possibility that it is not sufficiently delivered into the skin. Therefore, in order to prepare the molecular association prepared in Example 1 as a formulation applicable to the skin, a gel-type formulation was prepared to further include 0.7% by weight of hyaluronic acid (HA) and 2% by weight of 1,2-hexanediol in each of 0.04% by weight and 0.08% by weight of the molecular association of Example 1. The test results were confirmed in the confirmation of cytotoxicity of preadipocyte and differentiated adipocyte in Test Example 3 described later.

### Comparative Example 1. Precursor of deoxycholic acid

Deoxycholic acid itself, which did not pass through the silica in Example 1, was used.

### [Test Example]

### Test Example 1. Measurement of particle size of deoxycholic acid molecular association

The solubility of deoxycholic acid in water is very low at 0.024%. Therefore, in order to improve solubility and to be stable in aqueous phase, previously, in Example 1, through the process of the present invention, which involves dissolving the deoxycholic acid in ethanol, adding water and removing ethanol, the molecular association of deoxycholic acid in a colorless, odorless, and transparent liquid form in which deoxycholic acid molecules form a cluster is prepared. The particle size of 1.6 nm was confirmed through Zetasizer and TEM analysis for the molecular association of deoxycholic acid.

### Zetasizer analysis

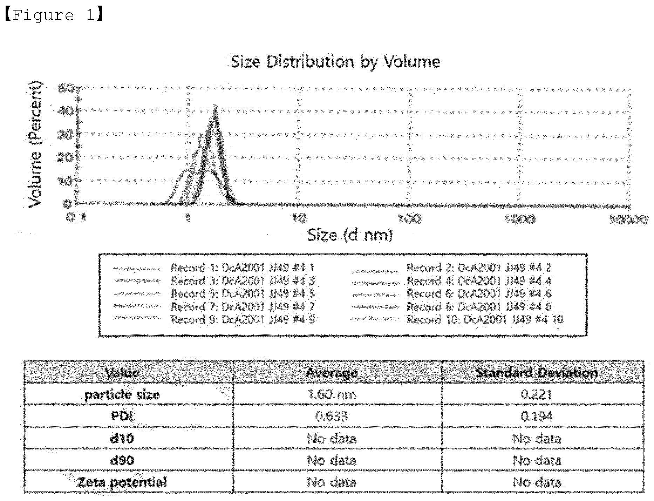

After filtering the deoxycholic acid molecular association of Example 1 with a 0.2 um filter, the particle size was measured 10 times using a Zetasizer (Malvern, Nano ZSP) under the conditions in Table 1 below, and the average particle size of 1.6 nm was confirmed from the size distribution by volume (Fig. 1).

**[Table 1]**

| | | | |
|---|---|---|---|
| **Temperature** | 25°C | **Duration Used** | 80 S |
| **Count rate** | 461.9 Kcps | **Measurement (set)** | 10 |
| **Cell Description** | Disposable sizing cuvette | **Attenuator** | 8 |

### TEM analysis

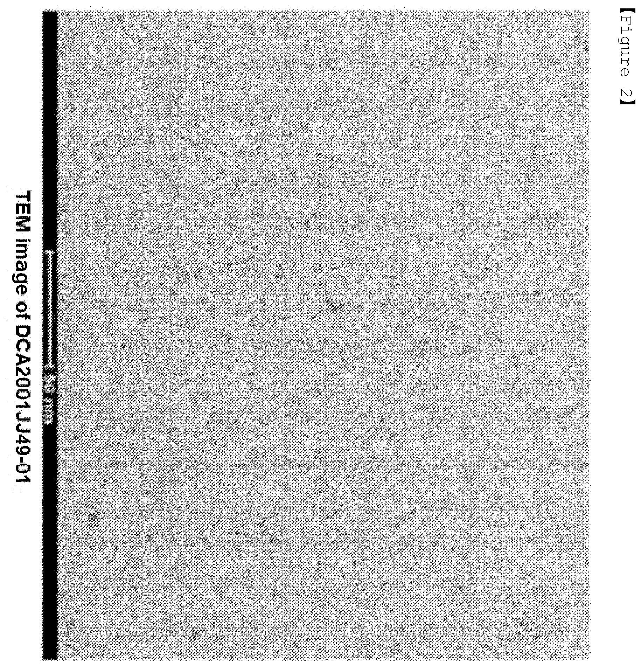

An EMS company's FCF300-Cu 50/pk (Formvar/Carbon 300Mesh, Copper) grid was placed on a filter paper, and about 20 µl of the sample was dropped on it with a micropipette. The grid was dried by waiting for 15 minutes or more without negative staining, and the particle size of the deoxycholic acid molecular association of Example 1 was measured by TEM image, and it was confirmed that it was similar to the particle size measured by Zetasizer (Fig. 2).

**[Table 2]**

| **Point resolution** | **Line resolution** | **HR STEM resolution** | **EDS resolution** |
|---|---|---|---|
| 0.205 nm | 0.102 nm | 0.16 nm | 136 eV |

### Test Example 2. Stability test

### (1) Materials

In order to confirm the storage stability of the molecular association of deoxycholic acid to which the present invention was applied, 292 g of DCA200265-67 without any change in physical properties was prepared in the same manner as in Example 1 and used as a stability test sample for 12 months.

**[Table 3]**

| **Batch No.** | **Sample Amount (g)** | **Conc. (%)** | **Particle Size (nm)** | **pH** | **Section** |
|---|---|---|---|---|---|
| DCA2002JJ65 | 98 | 1.92 | 1.6 | 9.16 | Stability test sample |
| DCA2002JJ66 | 96 | 2.35 | 1.9 | 9.13 | |
| DCA2002JJ67 | 98 | 2.28 | 1.6 | 9.16 | |

### ① Sample storage method

After taking 1.7 mL of the sample and putting it in one SciLab^{®}, SL/Vi1361 5 mL serum vial, the storage cap was closed, and the vial and storage cap were fixed using a capper. It was wrapped using para-film between the vial and the storage cap and labeled (including material name, batch No., test date).

### (2) Analysis method

### Property

Visually observed.

### Concentration

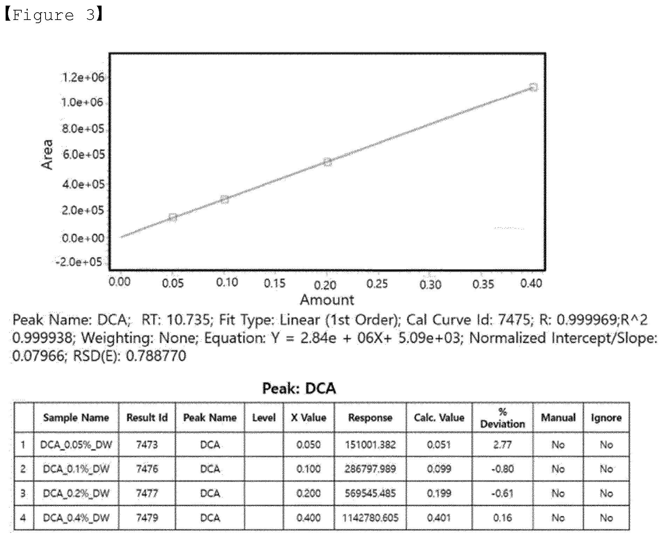

For concentration analysis, HPLC analysis was performed as shown in Table 4 below, and the resulting deoxycholic acid calibration curve is shown in Fig. 3.

**[Table 4]**

| | |
|---|---|
| **Column** | RP C18 (215x4.6) |
| **Mobile phase** | Water: ACN : 85% H₃PO₄ (50:50:0.1) |
| **Flow rate** | 1.0 mL/min |
| **Injection volume** | 10 µL |
| **Run time** | 17 min |
| **Wavelength** | 195 nm |
| **Sample Temp.** | 30 °C |
| **Column Temp.** | 25 °C |

### Particle size analysis

It was analyzed according to the Zetasizer analysis conditions and method of Example 1.

### pH

The molecular association of deoxycholic acid was filtered through a 0.45 um filter and measured using a Mettler toledo S220.

### ③ Result of accelerated stability test

### Property

It is a colorless and transparent liquid, and the same properties were maintained under 40±2°C/75±5% acceleration conditions for 12 months.

### Concentration

The change in concentration under accelerated conditions for 12 months is shown in Table 5 below, and no significant change in concentration was observed. Specifically, the change in concentration was measured in units of 1 month under accelerated conditions with temperature and humidity of 4 40±2°C/75±5% for 12 months, and the results are shown in Table 5 below. After calculating the difference between the initial (0 month) concentration and the subsequent concentration, the value divided by the initial concentration was calculated as the change rate of the concentration, and the average value of this change rate for 3 months and 12 months was calculated, respectively. It is described in Table 5 below.

**[Table 5]**

| | DCA2022JJ65 | DCA2022JJ66 | DCA2022JJ67 |
|---|---|---|---|
| 0 month | 1.88% | 1.92% | 1.93% |
| 1 month | 1.93% | 1.98% | 1.95% |
| 2 month | 1.920 | 1.99% | 1.99% |
| 3 month | 2.03% | 1.95% | 2.02% |
| 6 month | 1.900 | 1.98% | 2.02% |
| 9 month | 2.02% | 2.02% | 2.05% |
| 12 month | 2.05% | 2.05% | 2.10% |
| 3 month average rate of change | 4.26% | 2.78% | 2.94% |
| 12 month average rate of change | 5.05% | 3.91% | 4.75% |

### Particle size

Changes in particle size under accelerated conditions for 12 months are shown in Table 6 below, and there was no significant change in particle size. Specifically, the change in particle size was measured in units of 1 month under accelerated conditions with a temperature and humidity of 40±2°C/75±5% for 12 months, and the results are shown in Table 6 below. After calculating the difference between the particle size at the beginning (0 month) and the particle size thereafter, the value divided by the initial particle size was calculated as the change rate of the particle size, and the average value of this rate of change for 3 months and 12 months was calculated, respectively. The calculated values are listed in Table 6 below.

**[Table 6]**

| | DCA2022JJ65 | DCA2022JJ66 | DCA2022JJ67 |
|---|---|---|---|
| 0 month | 1.48 | 1.92 | 1.46 |
| 1 month | 1.54 | 1.99 | 1.49 |
| 2 month | 1.56 | 1.95 | 1.52 |
| 3 month | 1.60 | 1.97 | 1.54 |
| 6 month | 1.55 | 2.02 | 1.52 |
| 9 month | 1.62 | 2.05 | 1.58 |
| 12 month | 1.58 | 2.06 | 1.57 |
| 3 month average rate of change | 5.86% | 2.60% | 3.88% |
| 12 month average rate of change | 6.42% | 4.51% | 5.25% |

### pH

Table 7 below shows the pH change under accelerated conditions of 12 months, and showed stable pH without change over time. Specifically, the change in pH was measured in units of 1 month under accelerated conditions with temperature and humidity of 40±2°C/75±5% for 12 months, and the results are shown in Table 7 below. After calculating the difference between the pH value at the beginning (0 month) and the pH value thereafter, the value divided by the initial pH value was calculated as the change rate of the pH value, and the average value of this change rate for 3 month and 12 month was calculated, respectively. The calculated values are listed in Table 7 below.

**[Table 7]**

| | DCA2022JJ65 | DCA2022JJ66 | DCA2022JJ67 |
|---|---|---|---|
| 0 month | 9.15 | 9.13 | 9.15 |
| 1 month | 8.96 | 9.13 | 8.97 |
| 2 month | 9.03 | 9.23 | 9.14 |
| 3 month | 9.11 | 9.14 | 9.16 |
| 6 month | 9.20 | 9.31 | 9.27 |
| 9 month | 9.32 | 9.31 | 9.29 |
| 12 month | 9.27 | 9.36 | 9.33 |
| 3 month average rate of change | -1.280 | 0.40% | -0.66% |
| 12 month average rate of change | -0.02% | 1.280 | 0.47% |

### (1) Final result of accelerated stability test

The final results of the accelerated stability test were summarized and listed in Table 8 below.

**[Table 8]**

| **Test type** | **Test Condition** | **Test Interval** | **Test Item** | **Result** |
|---|---|---|---|---|
| Acceleration test | 40 ± 2°C 75 ± 50 | 0∼12 month | Property, concentration, Particle size, pH | No change over time |

As shown in Table 8, as a result of the accelerated stability test in three batches over a period of 12 months, no change over time was found in all test items and it was stable.

### Test Example 3. Confirmation of cytotoxicity of preadipocytes and adipocytes

### (1) Test material

To compare the cytotoxicity of preadipocytes and differentiated adipocyte, deoxycholic acid and deoxycholic acid molecular association (DCA2001JJ43, DCA2001JJ83-1) and deoxycholic acid salt molecular association (DCA2001JJ85-1) to which the present invention was applied were used.

### (2) Preadipocyte cytotoxicity

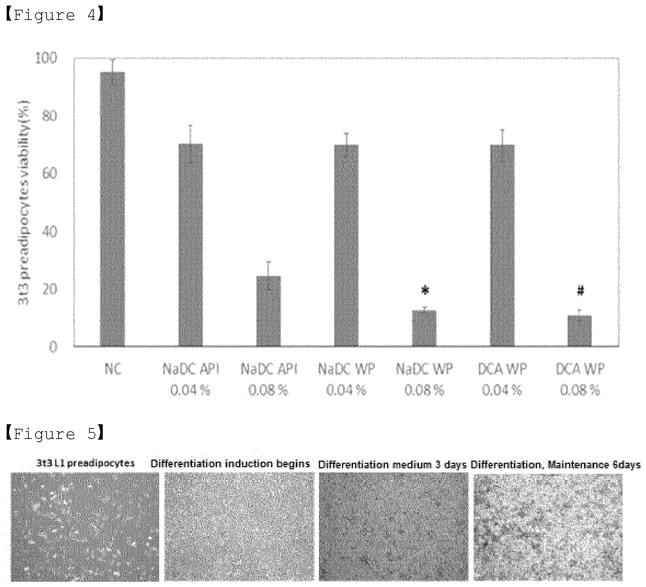

3T3-L1 preadipocytes were inoculated into a 96 well plate at 5×10³ per well, grown in medium (high glucose DMEM, 10% bovine calf serum, 1% penicillin/streptomycin) for 16 hours. The drug was prepared to contain 0.04% and 0.08%, respectively, and then treated for 4 hours. After adding 10 µl of Dojingo CK04-11 cell counting kit-8 to each well according to the manual, reacting for 2 hours, the absorbance at 450 nm was measured with a spectrophotometer to compare preadipocyte cytotoxicity. The results are shown in Fig. 4.

As a result, at 0.04%, the preadipocyte cytotoxicity of the precursor of deoxycholic acid salt and the molecular association of deoxycholic acid and the molecular association of deoxycholic acid salt were the same. However, when treated with 0.08%, compared to 24.6% of the precursor, the survival rate of preadipocytes to which the molecular association of deoxycholic acid salt was added decreased by 11.9% to 12.7% (*p=0.008), and in the group treated with the molecular association of deoxycholic acid, the survival rate decreased by 13.8% to 10.8% (#p=0.01). Therefore, the increase in preadipocyte cytotoxicity of the molecular association of deoxycholic acid to which the technology of the present invention was applied was confirmed.

### ③ Cytotoxicity of differentiated adipocyte

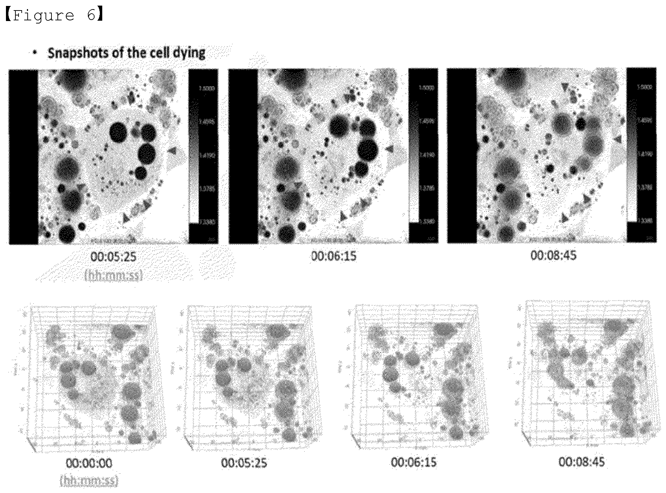

According to the Biovision 3T3 L1 differentiation kit manual, 3t3 L1 preadipocytes were grown to 100% confluence in a culture dish, and the culture medium was replaced with a differentiation maintenance medium. After 6 days, differentiated adipocyte with a large number of lipid droplets were induced (Fig. 5). Differentiated adipocytes were treated with 0.07% molecular association of deoxycholic acid(DCA2001JJ43). After acquiring 3D images at 2.5 frames per second using a Tomocube HT-2H microscope and applying the multi point acquisition function using imaging software TomoStudio, the changes in differentiated adipocytes were photographed at 25 second intervals for 40 minutes. As a result, the cell membrane and intracellular structure of the differentiated adipocyte, which were observed up to 5 minutes after imaging, rapidly faded after 5 minutes, and after 8 minutes, the RI value around the lipid droplet became almost the same as that of the external medium. From this, differentiated adipocyte cytotoxicity was confirmed (Fig. 6).

### Test Example 4. Preclinical trial

### ① SD rat skin penetration test

### Materials

An experiment was conducted to confirm the skin permeability and subcutaneous adipose tissue removal ability of the molecular association of deoxycholic acid (DCA2002JJ84 prepared by the method of Example 1) in SD rats.

### Test Method

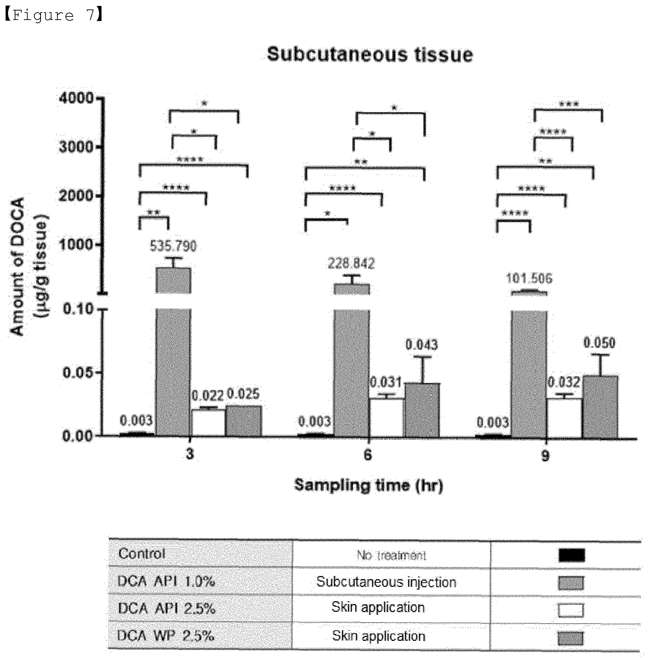

The interscapular region (back of the neck near the ear) of 10-week-old SD rats (280-350 g) with sufficient subcutaneous adipose tissue was shaved and anesthetized. A donor cell was attached to the shaved area with tape. 2.5% deoxycholic acid as a precursor and 500 µl of the deoxycholic acid molecular association of the present invention were added to the donor cells at 2.5%, and then applied to the skin continuously for 3, 6, and 9 hours under anesthesia. The distribution amount of the deoxycholic acid in subcutaneous tissue, skin, and plasma was checked to confirm skin permeability, and the cytotoxicity was confirmed in the subcutaneous fat cell layer through tissue staining, and the results are shown at the bottom of FIG. 7. A subcutaneous injection group of deoxycholic acid was used as a positive control group (1% DCA, 500 µl subcutaneous injection).

### Results of DCA distribution measurement in subcutaneous tissue

When the deoxycholic acid precursor and deoxycholic acid molecular association were continuously applied to the skin of SD rats, In both groups, an increase in skin permeability over time was confirmed by quantification of deoxycholic acid in the subcutaneous tissue. In the subcutaneous injection group, the amount of DCA gradually decreased in the subcutaneous tissue after subcutaneous injection, whereas in the skin application group, it increased over time, and the increase in transmittance of the deoxycholic acid molecular association was slightly higher than that of the deoxycholic acid precursor (Fig. 7).

### Result of measuring the amount of DCA distribution in the skin

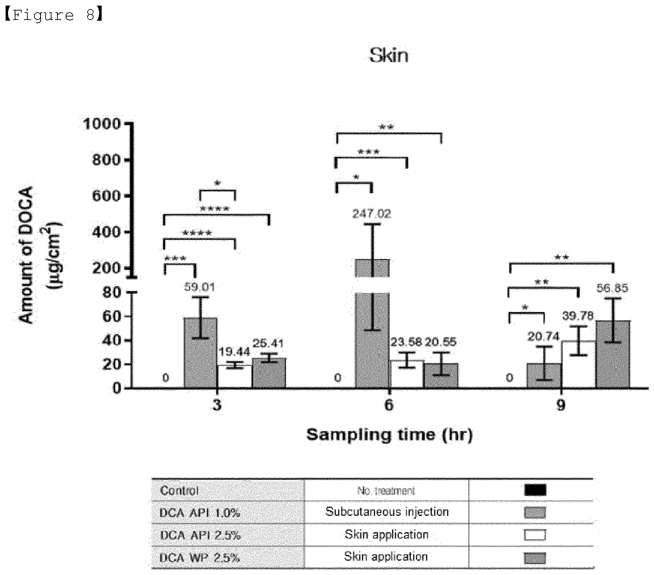

When the deoxycholic acid precursor and deoxycholic acid molecular association were continuously applied to the skin of SD rats, an increase in the deoxycholic acid distributed over the skin was confirmed in both groups over time. On the other hand, the subcutaneous injection group had the highest distribution of deoxycholic acid in the skin tissue after 6 hours, but showed a lower distribution than the skin application group at 9 hours (Fig. 8).

### Results of DCA distribution measurement in plasma

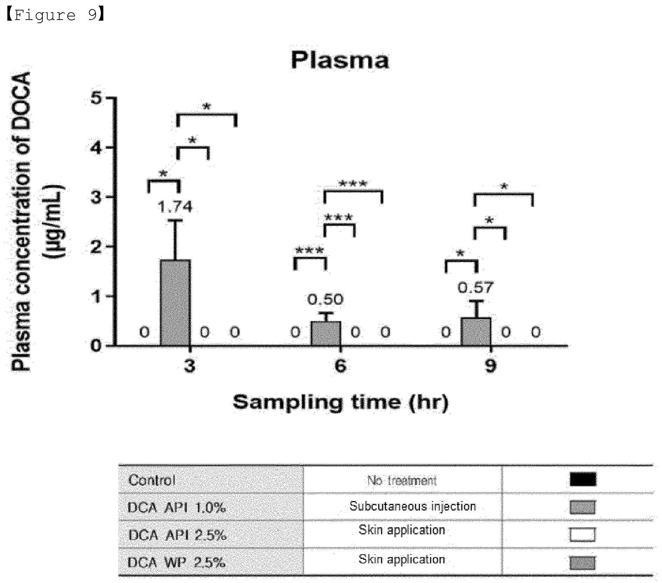

Fig. 9 shows the concentration of DCA in rat plasma. DCA was detected in plasma only in the deoxycholic acid subcutaneous injection group, but not in the skin application group. From this, it was confirmed that the deoxycholic acid molecular association has a subcutaneous fat removal effect by skin application, but does not transfer to plasma, so there is no safety problem.

### Results of histological analysis

500 µL of 1% deoxycholic acid was subcutaneously injected, and 500 µL of 2.5% deoxycholic acid and 500 µL of 2.5% deoxycholic acid molecular association were applied under the skin where the franz cells were attached, respectively, and applied continuously for 3, 6, and 9 hours. Then, tissues (skin, subcutaneous tissue) of the interscapular region were collected, fixed, and subjected to H&E staining and Masson's trichome staining. Tissue changes were investigated through microscopic observation.

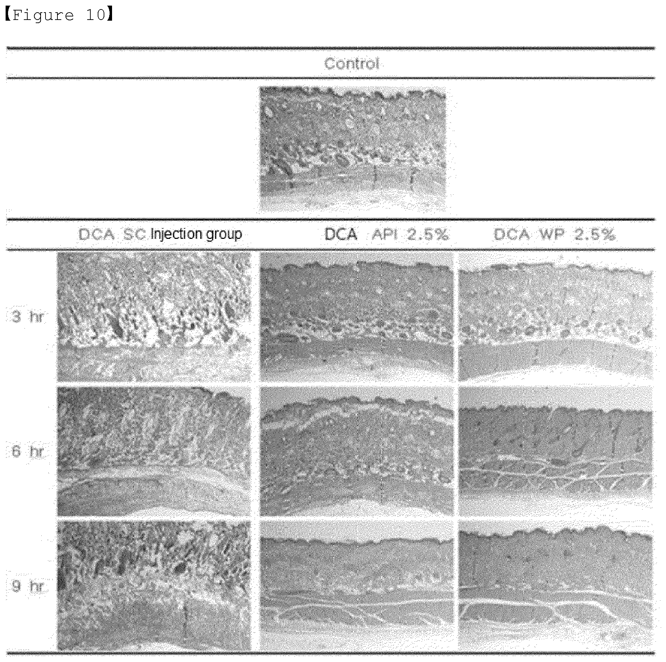

As a result of H&E staining, in the subcutaneous injection group of deoxycholic acid, severe tissue damage was observed after subcutaneous injection. In the skin application group, a decrease in dermis white adipose tissue was observed over time, and this was more clearly observed in the molecular association of deoxycholic acid application group (Fig. 10).

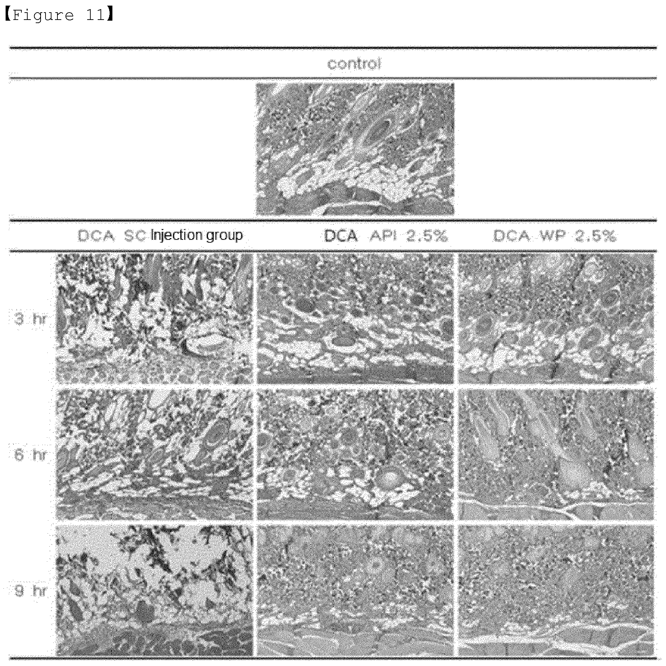

As a result of Masson's trichome staining to observe the increase or decrease of collagen in the skin and subcutaneous tissue, an increase in the area stained blue due to destruction of fat cells or increased collagen was observed in the skin application group (Fig. 11). In the subcutaneous injection group of deoxycholic acid, hematoma and edema occurred in the tissue, and the tissue was hardened during section creation due to severe tissue damage. Adequate tissue sections were not obtained in the 9 hr treatment group because the sections were split due to adipose tissue hardening.

### Conclusion

In the skin application group, the amount of deoxycholic acid distributed in the skin and subcutaneous tissue increased with time. The molecular association of deoxycholic acid showed a higher amount of deoxycholic acid distributed in the skin or subcutaneous tissue compared to the group treated with the precursor of deoxycholic acid. The concentration of deoxycholic acid in plasma was detected only in the subcutaneous injection group, and in the histological analysis, a decrease in adipose tissue was observed in the skin application group over time after administration. This phenomenon appeared more clearly in the molecular association of deoxycholic acid treatment group. As a result of the pharmacokinetic study, the amount of deoxycholic acid distributed in the subcutaneous tissue accumulated over time, and in histological analysis, the dermis white adipose tissue in the skin decreased. Therefore, it can be concluded that the molecular association of deoxycholic acid effectively penetrated the skin and contributed to the reduction of adipose tissue.

### ② Lipolysis effect through skin penetration using ob/ob obese mouse model

### Material

2.52% molecular association of deoxycholic acid was prepared in the same manner as in Example 1 (DCA2103JJ134-02, 115 g, 95% recovery rate), diluted to 1.0% and 2.5% for ob/ob obese mouse fat reduction efficacy evaluation, and formulated (1.0% SCAI-101, 2.5% SCAI-101).

### Test method

Using the ob/ob obese mouse model, the molecular association of deoxycholic acid (1.0% SCAI-101, 2.5% SCAI-101) prepared in Example 1 was tested to confirm the fat reduction effect after skin penetration. Hair was removed from the back and abdomen of an ob/ob obese mouse, and 100 µL of a solution (SCAI-101) containing 1.0% and 2.5% molecular association of deoxycholic acid, respectively, was applied to two sites (total of four sites) of 1cm² size, twice a day, to be sufficiently absorbed. Changes in body weight, waist girth, and abdominal fat mass obtained by micro-CT imaging of the mouse abdomen for 4 weeks were compared with the negative control group, and skin permeability and lipolysis efficacy were confirmed as shown in Table 9.

**[Table 9]**

| **Test group** | **Test material** | **Number of animals** |
|---|---|---|
| Group 1 | Control (Vehicle) | 5 |
| Group 2 | 1.0% SCAI-101 application group | 5 |
| Group 3 | 2.5% SCAI-101 application group | 5 |

### Body weight change

The body weight of the ob/ob obese mouse control group continued to grow while consuming normal feed, and increased from 46.2±1.13g (Week 0) to 51.1±0.89g (Week 4) for 4 weeks. In comparison, there was a statistically significant increase at Week 3 and Week 4 (p<0.01 and p<0.001, respectively). In the case of the 2.5% concentration application group, body weight also increased from 43.4±1.62g (Week 0) to 48.3±1.49g (Week 4), and a statistically significant increase was observed at week 4 compared to the body weight at Week 0. (p<0.05). However, in the 1.0% concentration application group, body weight decreased from 42.1±2.14g (Week 0) to 41.714.07g (Week 4).

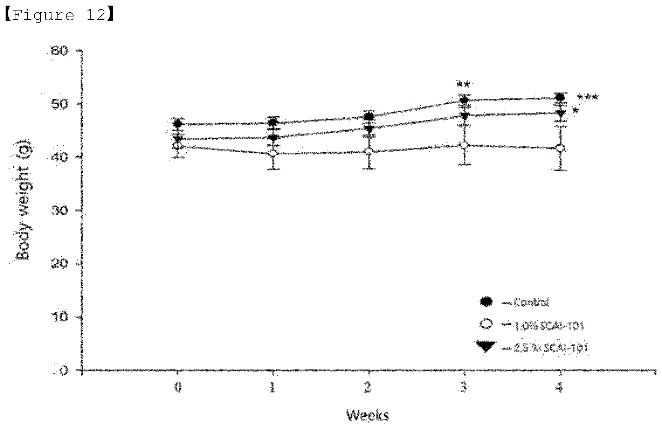

As shown in Tables 10 below and Fig. 12, it was confirmed that the body weight of the control group and the 2.5% application group increased continuously, and when comparing the body weight change between the 1.0% and 2.5% application groups, no dose-dependent tendency was observed.

**[Table 10]**

| **Weeks** | **Control** | 1.0% SCAI-101 application group | 2.5% SCAI-101 application group |
|---|---|---|---|
| 0 | 46.211.13 | 42.112.14 | 43.411.62 |
| 1 | 46.5±1.08 | 40.7±2.83 | 43.7±1.52 |
| 2 | 47.6±1.09 | 41.0±3.12 | 45.5±1.65 |
| 3 | 50.7±0.99** | 42.3±3.64 | 47.8±1.61 |
| 4 | 51.1±0.89*** | 41.7±4.07 | 48.3±1.49* |
| Data were expressed as mean ± SEM. | | | |
| The weight of each group was measured weekly for 4 weeks. Paired t-test was used for the comparison of the body weight of the week 0. *, ** and ***: Statistically significant compared with Week 0 (before treatment) (p<0.05, p<0.01 and p<0.001, respectively) | | | |

### Waist measurement and change in waist measurement

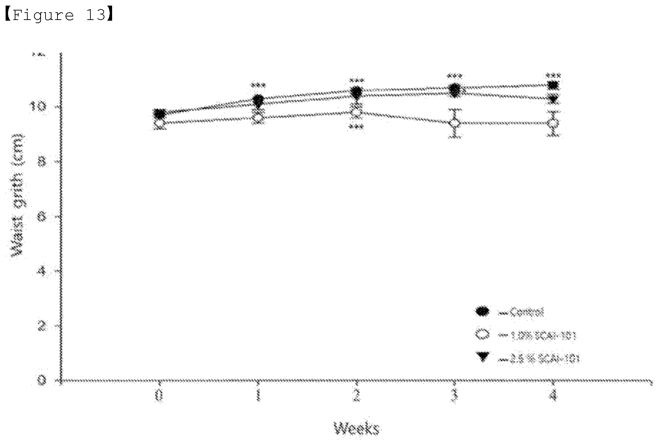

As a result of measuring waist girth by applying twice a day for 4 weeks to ob/ob obese mice, the waist girth of the control group increased statistically significantly after 1, 2, 3 and 4 weeks compared to week 0. (all p<0.001). (Fig. 13 and Table 11)

**[Table 11]**

| **Weeks** | **Control** | **1.0% Conc. application group** | **2.5% Conc. application group** |
|---|---|---|---|
| 0 | 9.7±0.11 | 9.4±0.21 | 9.8±0.14 |
| 1 | 10.3±0.05*** | 9.6±0.24# | 10.1±0.19 |
| 2 | 10.6±0.09*** | 9.8±0.25***, # | 10.4±0.26 |
| 3 | 10.7±0.12*** | 9.4±0.50# | 10.5±0.13* |
| 4 | 10.8±0.13*** | 9.4±0.43# | 10.3±0.16 |
| Data were expressed as mean ± SEM. | | | |
| The waist girth of each group was measured weekly for 4 weeks. Paired t-test was used for the comparison of the waist girth of the Week 0. * and ***: Statistically significant compared with Week 0 (before treatment) (p<0.05 and p<0.001, respectively). #: | | | |
| Statistically significant compared with the same week of Control (p<0.05 and p<0.01, respectively). Ordinary ANOVA test, followed by Tukey multiple comparison post hoc test, was used for the comparison of the waist girth (Week 1, Week 3 and Week 4). | | | |
| Kruskal-Wallis test, followed by Dunn's multiple comparison test, was used for the comparison of the waist girth (Week 2). | | | |

When the molecular association of deoxycholic acid was applied, in the case of 1.0% concentration, the waist girth increased slightly compared to Week 0, showing a statistically significant increase at Week 2 (p<0.001), but decreased from Week 3 and remained similar to the level of the Week 0 without a statistically significant increase until Week 4. On the other hand, compared to the waist girth of the control group in the same week after application, in the case of the 1.0% concentration application group, the waist girth was statistically significantly shorter at Week 1, Week 2, Week 3 and Week 4 (all p<0.05) (Table 12). Consistent with this, the change in waist girth (waist girth gain) of the 1.0% concentration application group decreased statistically significantly at Weeks 1, 3, and 4 compared to the control group for the same week (all p<0.05) (Table 11). The waist girth of the 2.5% concentration application group was statistically significantly increased at Week 3 compared to the waist girth at Week 0. However, when compared with the control group in waist girth gain, statistical significance was not recognized, so it was judged to be a temporary result (Table 12).

**[Table 12]**

| **Weeks** | **Control** | **1.0% Conc. application group** | **2.5% Conc. application group** |
|---|---|---|---|
| 1 | 0.3±0.0 | -0.4±0.2* | 0.1±0.2 |
| 2 | 0.6±0.1 | -0.2±0.2 | 0.4±0.3 |
| 3 | 0.7±0.1 | -0.6±0.5* | 0.5±0.1 |
| 4 | 0.8±0.1 | -0.6±0.4* | 0.3±0.2 |
| Data were expressed as mean±SEM. | | | |
| The waist girth gain of each group was measured weekly for 4 weeks. ANOVA test, followed by Tukey multiple comparison post hoc test, was used for the comparison of the waist girth gain. *: Statistically significant compared with girth gain of every week of Control (p<0. 05) . | | | |

### Abdominal fat mass measurement result using micro-CT scan

As a result of measuring the fat mass around the waist and the abdomen (between 3-5 lumbar vertebrae) including the application site of the test substance, The subcutaneous fat mass of the 1.0% and 2.5% concentration application groups were 2233.21351.4mm³ and 2589.7193.4mm³, respectively, which were not statistically significant when compared to the control group (2505.41279.4mm³), and the percentage of subcutaneous fat mass was 89.1114.03% and 103.413.73%, respectively, compared to the control group. The subcutaneous fat mass of the 1.0% concentration application group was 10.9% lower on average than the control group, but no dose-dependent decrease was observed compared to the 2.5% concentration application group. (Table 13)

**[Table 13]**

| **Item** | **Region of Interest** | **Control** | **1.0% Conc. application group** | **2.5% Conc. application group** | **Unit** |
|---|---|---|---|---|---|
| Objective | Subcutaneous | 2505.4±279.4 | 2233.2±351.4 | 2589.7±93.4 | |
| Vol. of adipose tissue | Visceral | 3861.5±289.9 | 3081.2±571.0 | 3475.2±108.4 | mm³ |
| % Fat volume | Subcutaneous | 100.0±11.15 | 89.1±14.03 | 103.4±3.73 | % |
| | Visceral | 100.0±7.51 | 79.8±14.79 | 90.0±2.81 | |
| Data were expressed as mean±SEM (n=3) | | | | | |

### Evaluation of adipose tissue thickness of applied skin tissue

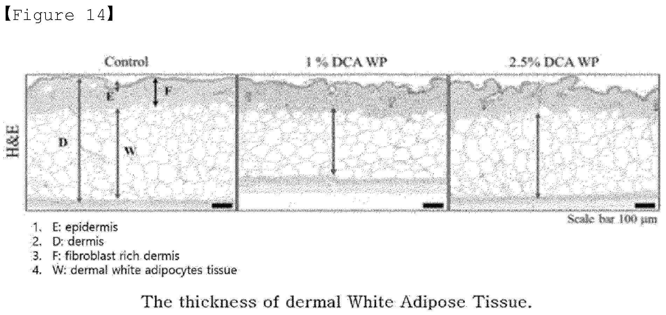

The thickness of the adipocyte tissue of the skin to which the molecular association of deoxycholic acid (SCAI-101, DCA WP) prepared in Example 1 was directly applied was measured. As a result, in the control group, and the 1.0% and 2.5% concentration application groups, the thickness was 594.6±30.46*µ*m, 394.3±17.96*µ*m and 492.4±11.42*µ*m respectively at Week 4 after application twice a day. In both the 1.0% and 2.5% concentration application groups, there was a statistically significant decrease when compared to the control group (all p<0.05). The ratio (% Average) of the adipose tissue thickness of the control group to the adipose tissue thickness of the groups applied with the molecular association of deoxycholic acid was 66.012.94% (1.0% concentration application group) and 82.6±1.87% (2.5% concentration application group), respectively, and a statistically significant decrease was confirmed only in the low concentration 1.0% concentration application group (p<0.05). It was confirmed that the lipolysis and reduction of accumulation of the 1.0% concentration application group was superior to that of the 2.5% concentration application group. (Fig. 14 and Table 14)

**[Table 14]**

| **Group** | **Thickness (*µ*m)** | **% Average** |
|---|---|---|
| Control | 594.6130.46 | 100±4.99 |
| 1.0% Conc. application group | 394.3±17.96* | 66.0±2.94* |
| 2.5% Conc. application group | 492.4±11.42* | 82.6±1.87 |
| Data were expressed as mean ± SEM (n=20) | | |
| Kruskal-Wallis test, followed by Dunn's multiple comparison test, was used for the comparison of the thickness of Dermal White Adipose Tissue. *: Statistically significant compared with Control (Vehicle treatment) . | | |

### Results

After applying the 1.0% concentration application group (1.0% SCAI-101, DCA WP) twice a day for 4 weeks, statistically significant reductions in waist girth and adipose tissue thickness were confirmed. From this, the pharmacological effect of reducing or decomposing the amount of skin fat after skin penetration was confirmed. These results can be attributed to the skin-permeable pharmacological action of the molecular association of deoxycholic acid directly applied to the skin, unlike the characteristics of deoxycholic acid, which is generally known to be difficult to penetrate the skin.

Compared with the control group, the reduction in body weight, waist girth, and subcutaneous fat mass was predicted to be the result of an indirect effect by the direct adipocyte decomposition of the molecular association of deoxycholic acid permeated into the skin.

Therefore, it is judged that the skin permeable lipolytic effect of the 1.0% concentration application group, which is the condition of the present invention, was the most appropriate concentration. However, the effect of the 2.5% concentration application group and the dose-dependent tendency were not recognized, but in the future, through appropriate concentration and composition improvement, a solution containing the molecular association of deoxycholic acid will be able to maximize the lipolysis effect.

## Claims

1. A molecular association in which bile acid molecule or bile salt molecules are physically bonded,
wherein when the molecular association is formed in a composition containing water,
the molecular association has an associated structure in the composition.

2. The molecular association according to claim 1, wherein the average particle diameter of the molecular association is 1.0 to 10 nm or less.

3. The molecular association according to claim 1, wherein the molecular association is amorphous.

4. The molecular association according to claim 1, wherein the molecular association has the pH of exceeding 8.5 and less than 10.

5. The molecular association according to claim 1, wherein the molecular association has a concentration change rate of exceeding 1 and less than 10% for 12 months under 40±2°C/75±5% accelerated conditions.

6. The molecular association according to claim 1, wherein the molecular association has a particle size change rate of exceeding 1 and less than 10% for 12 months under 40±2°C/75±5% accelerated conditions.

7. The molecular association according to claim 1, wherein the molecular association has a pH change rate of exceeding 0 and less than 5% for 12 months under 40±2°C/75±5% accelerated conditions.

8. The molecular association according to claim 1, wherein the bile acid is any one from the group consisting of cholic acid, chenodeoxycholic acid, glycocholic acid, taurocholic acid, deoxycholic acid and lithocholic acid, and the bile salt is any one salt from the group consisting of cholic acid, chenodeoxycholic acid, glycocholic acid, taurocholic acid, deoxycholic acid and lithocholic acid.

9. A pharmaceutical composition for locally reducing fat comprising the molecular association of claim 1.

10. The pharmaceutical composition for locally reducing fat according to claim 9, wherein the composition is applied and used as an external skin preparation, which is a non-surgical and no injection method.

11. The pharmaceutical composition for locally reducing fat according to claim 9, wherein the composition further comprises at least one selected from the group consisting of glycerin, chia seed oil, glucan, hyaluronic acid, Lonicera Japonica (Honeysuckle) flower extract, collagen, ceramide, lecithin, betaine, trehalose, panthenol, squalane, caprylic/capric triglyceride, butylene glycol, propane diol, Pentylene glycol, Sodium Levulinate, Hydrogenated Lecithin, and Sodium Hyaluronate.

12. The pharmaceutical composition for locally reducing fat according to claim 9, wherein the composition comprises the molecular association in an amount of 0.05 to 10% by weight.

13. The pharmaceutical composition for locally reducing fat according to any one of claim 9 to claim 11, wherein the composition is **characterized in that** it is intended to treat a disease selected from the group consisting of obesity, fat redistribution syndrome, submental fat which is a double chin caused by local fat accumulation, lower eyelid fat herniation formation, lipomas, Dercum's disease, lipodystrophy, buffalo hump dystrophy and combinations thereof.

14. The pharmaceutical composition for locally reducing fat according to any one of claim 9 to claim 11, wherein the composition is **characterized in that** it is localized to a site selected from the group consisting of abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof.

15. The pharmaceutical composition for locally reducing fat according to any one of claim 9 to claim 11, wherein the composition is used in any one formulation from the group consisting of gel, cream, ointment, unguent, spray, thickened formulation and cataplasm.

16. The pharmaceutical composition for locally reducing fat according to any one of claim 9 to claim 11, wherein the composition has a bile acid concentration of 0.001 to 0.2 ug/ml measured in plasma 3 hours after application to the skin.
